Europäisches Patentamt

European Patent Office

Office européen des brevets

(11) Publication number: **0 437 001 A1**

# EUROPEAN PATENT APPLICATION

(21) Application number: 90203517.9

(51) Int. Cl.⁵: **C07C 69/675, C07C 67/26**

(22) Date of filing: 28.12.90

(30) Priority: 06.01.90 NL 9000035

(43) Date of publication of application:
17.07.91 Bulletin 91/29

(84) Designated Contracting States:
AT BE CH DE DK ES FR GB GR IT LI NL SE

(71) Applicant: STAMICARBON B.V.
Mijnweg 1
NL-6167 AC Geleen(NL)

(72) Inventor: Zonjee, Johanna Maria
St. Josephstraat 116
NL-5211 NJ Den Bosch(NL)

(54) Reaction product of an epoxidized fatty acid or its ester with an alpha,beta-unsaturated acid.

(57) The invention relates to the reaction product of an epoxidized fatty acid or its ester and an $\alpha,\beta$-unsaturated acid.

The percentage of esterified $\alpha,\beta$-unsaturated acid is at least 70%. The percentage of side-reactions is at most 20%.

EP 0 437 001 A1

# REACTION PRODUCT OF AN EPOXIDIZED FATTY ACID OR ITS ESTER WITH AN α,β-UNSATURATED ACID

The invention relates to a reaction product of an epoxidized fatty acid or its ester with an α,β-unsaturated acid.

Such a reaction product is known from US-A-3125592. This patentpublication describes a process for the reaction of epoxidized oils and α,β-unsaturated acids. A disadvantage of the described process is the occurrence of side reactions, such as the etherification of the oxirane groups with the OH groups formed and the polymerisation of the unsaturated bonds of the α,β-unsaturated acid or ester. The process according to US-A-3125592 results in a product, with a high molecular weight, which has to be purified to remove excess of one of the reactants, which is usually (meth)acrylic acid. Furthermore due to the high molecular weight and the broad range of products that is formed, the reaction mixture is difficult to process. The percentage of esterified α,β-unsaturated acid at reaction conditions involving equimolar amounts of epoxidized fatty acid or its ester and α,β-unsaturated ester is at most about 60%.

The object of the invention is to provide a reaction product of an epoxidized fatty acid or its ester with an α,β-unsaturated acid with a higher molar reactivity and consequently a higher percentage of acrylation and a lower percentage of side reactions.

The reaction product according to the invention is characterized in that the percentage of esterified α,β-unsaturated acid is at least 70%.

The percentage of esterified α,β-unsaturated acid is defined by:

$$\dfrac{A}{B} \times 100$$

where

A = the number of moles of α,β-unsaturated acid that has reacted via an acid-epoxy reaction and

B = the total number of moles of epoxy units used.

According to the present invention the percentage of side-reactions is at most 20%.

The percentage of side-reactions is defined by:

$$\left(1 - \dfrac{C}{D}\right) \times 100$$

where

C = the number of moles of α,β-unsaturated acid that has reacted via an acid-epoxy reaction and

D = the total number of moles of epoxy units consumed in the reaction.

The percentage of esterified α,β-unsaturated acid and the percentage of side reactions are based on reaction conditions involving substantially equimolar amounts of epoxy and acid.

In this manner a well processable product with high reactivity and a high percentage of esterification via an acid-epoxyreaction is obtained.

According to a preferred embodiment of the invention the percentage of esterified α,β-unsaturated acid is at least 75%.

Suitable α,β-unsaturated acids are, for example, unsaturated aliphatic acids such as acrylic acid, propiolic acid, methacrylic acid, crotonic acid, isocrotonic acid, oleic acid, elaidic acid, maleic acid, fumaric acid, citraconic acid, mesaconic acid. Other suitable acids are for example carbocylic carboxylic acids such as atropic acid and/or cinnamic acid. Preferably, use is made of acrylic acid and methacrylic acid. It is also possible to use hemiesters of unsaturated diacids, such as the monobutyl ester of maleic acid, as α,β-unsaturated acid.

Oils that are suitable as basis for the epoxidized oils are, for example, soya bean oil, linseed oil, safflower oil, caraway oil, rape oil, wood oil and/or fish oil. Preferably, use is made of soya bean oil and linseed oil. Natural epoxidized oils like vernomia oil are also suitable.

As alternative to oils, the esters of fatty acids with polyols other than glycerol may be used. Examples of suitable polyols are trimethylolpropane or (di-)pentaerythrytol. The acids of the aforementioned unsaturated oils and tall oil fatty acid may be used as fatty acids.

The process for the preparation of the reaction product according to the invention is characterized in that the α,β-unsaturated acid and the epoxidized fatty acid or its ester react at temperatures between 100°C and 130°C, in the presence of lithium hydroxide or chromium chloride.

Preferably, the temperature is between 115°C and 125°C.

The amount of lithium hydroxide or chromium chloride is usually between 0.01 and 5 wt.% (relative to the α,β-unsaturated acid).

By using lithium hydroxide or chromiumchloride the percentage of esterified α-β-unsaturated acid of at least 70% and the percentage of sidereaction of at most 20% is achieved. The per-

centage of esterified $\alpha,\beta$-unsaturated acid and the percentage of side reactions are based on reaction conditions involving substantially equimolar amounts of epoxy and acid.

US-A-3256225 describes also a process for reacting an epoxydized fatty acid and an $\alpha,\beta$-unsaturated acid. However, this process has the same disadvantages as already described for US-A-3125592.

Moreover, acids, such as acetic acid, may optionally be present during the reaction.

Suitable solvents for both the acid and the oil are, for example, xylene, toluene, butylacetate, cyclohexanone, dimethylformamide and hydrocarbon fractions.

The reaction product according to the invention can be used as one of the components of a multicomponent system such as for example in high solids binding agents where the reaction product can be combined as reactive diluent with an alkydresin, or for example in unsaturated polyester systems where, the reaction product is a substitute for styrene.

The reaction products according to the invention can also be used in UV- or electron-beam-curable or thermosetting systems suitable for printing inks, coatings or adhesives. In UV-curing paints the the reaction products according to the invention can be used as crosslinking agent together with ethylenic unsaturated monomers.

Possible applications are also use in formaldehyde-free chipboard adhesives or in coatings for fertilizers or wood.

The invention is illustrated with the following, non-limiting example.

Example I

145 parts by weight of acrylic acid were dissolved in 145 parts by weight of xylene, in which 0.84 parts by weight of lithium hydroxide and 0.75 parts by weight of monotertiary butylhydroquinone were dissolved. This solution was heated to 120° C. Over a period of 5 hours 750 parts by weight of a 50% solution of epoxidized linseed oil in xylene were added. After a reaction time of 8 hours an acid number of 48.8 and a weight per epoxy of 1874 were obtained and the experiment was stopped. The percentage of esterified acrylic acid was 76% and the percentage of side-reactions was 11%.

**Claims**

1. Reaction product of an epoxidized fatty acid or its ester with an $\alpha,\beta$-unsaturated acid, characterized in that the percentage of esterified $\alpha,\beta$-unsaturated acid is at least 70%.

2. Reaction product according to claim 1, characterized in that the percentage of esterified $\alpha,\beta$-unsaturated acid is at least 75%.

3. Reaction product according to claim 1 or 2, characterized in that the $\alpha,\beta$-unsaturated acid is acrylic acid or methacrylic acid.

4. Reaction product according to any one of claims 1-3, characterized in that the epoxidized fatty acid or its ester comprises soya bean oil or linseed oil.

5. Reaction product to any one of claims 1-4, characterized in that the percentage of side-reactions is at most 20%.

6. Process for the preparation of a reaction product according to any one of claims 1-5, characterized in that the $\alpha,\beta$-unsaturated acid and the epoxidized fatty acid or its ester react at temperatures between 100° and 130° C, in the presence of lithium hydroxide or chromium chloride.

7. Use of a reaction product according to any one of claims 1-5 or use of a reaction product obtained according to claim 6.

8. Curable system based on a reaction product according to any one of claims 1-5 or based on a reaction product obtained according to claim 6.

Claims for the following Contracting State: ES

1. Process for the preparation of a reaction product of an epoxidized fatty acid or its ester with an $\alpha,\beta$-unsaturated acid characterized in that the $\alpha,\beta$-unsaturated acid and the epoxidized fatty acid or its ester react at temperatures between 100° and 130° C, in the presence of lithium hydroxide or chromium chloride and that the percentage of esterified $\alpha,\beta$-unsaturated acid is at least 70%.

2. Reaction product according to claim 1, characterized in that the percentage of esterified $\alpha,\beta$-unsaturated acid is at least 75%.

3. Reaction product according to claim 1 and 2, characterized in that the percentage of side-reactions is at most 20%.

4. Reaction product according to any one of claims 1-3, characterized in that the $\alpha,\beta$-unsaturated acid is acrylic acid or methacrylic acid.

5. Reaction product according to any one of claims 1-4, characterized in that the epoxidized fatty acid or its ester comprises soya bean oil or linseed oil.

European
Patent Office

EUROPEAN SEARCH
REPORT

Application Number

EP 90 20 3517

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 256 225   (CH.S. NEVIN)(14-06-1966)<br>* Column 1, line 40 - column 2, line 44; column 3, lines 53-58; column 4, lines 31-45; column 5, lines 1-32; example 1; column 6, example 3; column 7, examples 7,8 *<br>– – – | 1-8 | C 07<br>C 69/675<br>C 07 C 67/26 |
| X | FR-A-2 029 438   (CONTINENTAL CAN CO.)(23-10-1970)<br>* Page 4, line 19 - page 5, line 3; page 5, line 22 - page 6, line 30; pages 9-10, example; pages 13-40, claims 1,2,5,13,14 *<br>– – – – – | 1-8 | |

|  |  |
|---|---|
|  | TECHNICAL FIELDS SEARCHED (Int. Cl.5)<br><br>C 07 C 69/00<br>C 07 C 67/00 |

The present search report has been drawn up for all claims

| Place of search | Date of completion of search | Examiner |
|---|---|---|
| The Hague | 19 March 91 | KINZINGER J.M. |

CATEGORY OF CITED DOCUMENTS

X: particularly relevant if taken alone
Y: particularly relevant if combined with another
   document of the same catagory
A: technological background
O: non-written disclosure
P: intermediate document
T: theory or principle underlying the invention

E: earlier patent document, but published on, or after
   the filing date
D: document cited in the application
L: document cited for other reasons

&: member of the same patent family, corresponding
   document